# EUROPEAN PATENT APPLICATION

(11) **EP 4 455 989 A1**
(43) Date of publication of application: **30.10.2024**
(21) Application number: 24172731.2
(22) Date of filing: 26.04.2024
(51) Int. Cl.: G06T 5/50, G06T 5/60

(54) **METHOD AND APPARATUS FOR GENERATING IMAGE RECONSTRUCTION MODEL AND METHOD AND APPARATUS FOR RECONSTRUCTING IMAGE USING IMAGE RECONSTRUCTION MODEL**

(30) Priority: 26.04.2023 KR 20230055001
(71) Applicant: Medicalip Co., Ltd., Gangwon-do 24341 (KR)
(72) Inventor: PARK, Sang Joon, Seoul (KR); KIM, Jong Min, Yongin-si, Gyeonggi-do (KR); LEE, Da In, Seoul (KR)
(74) Representative: Patentanwälte Ruff, Wilhelm, Beier, Dauster & Partner mbB

(57) **Abstract**

Provided are a method and apparatus for generating an image reconstruction model and an image reconstruction method and apparatus using the image reconstruction model. The image reconstruction apparatus extracts, through an encoder configured to extract a structure and a texture, a structure and a texture of each of a first medical image and a second medical image and generates, through a decoder configured to reconstruct an image based on a structure and a texture, a third medical image in which the texture of the second medical image is combined with the structure of the first medical image.

## Description

### [Technical Field]

An embodiment of the present disclosure relates to a method and apparatus for generating a model for reconstructing a medical image and a method and apparatus for reconstructing a medical image by using the model.

### [Background Art of the Invention]

In medical images, brightness values, etc., may vary depending on the photographing equipment and photographing environment. For example, if photographing is performed with the same X-ray equipment, a brightness value in an X-ray image may vary with brightness of ambient lighting. Alternatively, if different X-ray equipment is used even for photographing in the same environment, brightness values in two X-ray images may be different from each other. It is impossible to convert medical images having brightness values varying with the characteristics of the photographing equipment or surrounding environment so as to appear as images captured with the same machine in the same environment by adjusting the medical images using an existing image processing method.

### [Disclosure of the Invention]

An embodiment of the present disclosure aims to provide a method and apparatus for generating an image reconstruction model for reconstructing an image by converting a texture while maintaining a structure in a medical image.

An embodiment of the present disclosure also aims to provide a method and apparatus for reconstructing a texture of a medical image by using the image reconstruction model.

### [Means for Solving the Problems]

To achieve the foregoing technical objects, as an example of an image reconstruction method according to an embodiment of the present disclosure, a method, performed by an image reconstruction apparatus, of reconstructing an image, includes extracting, through an encoder configured to extract a structure and a texture from an image, a first structure and a first texture from a first medical image, extracting, through the encoder, a second structure and a second texture of a second medical image, and inputting the first structure of the first medical image and the second texture of the second medical image to a decoder configured to reconstruct an image based on a structure and a texture and generate a third medical image.

To achieve the foregoing technical objects, an example of a method of generating an image reconstruction model according to an embodiment of the present disclosure includes inputting a first medical image to an image reconstruction model including an encoder configured to extract a structure and a texture from an image and a decoder configured to reconstruct the image based on the structure and the texture and generate a second medical image, and primarily training the image reconstruction model based on a first loss function indicating an error between the first medical image and the second medical image.

To achieve the foregoing technical objects, an example of an image reconstruction apparatus includes a feature extraction unit configured to extract, through an encoder configured to extract a structure and a texture, a structure and a texture of each of a first medical image and a second medical image and a reconstruction unit configured to generate, through a decoder configured to reconstruct an image based on a structure and a texture, a third medical image in which the texture of the second medical image is applied to the structure of the first medical image.

### [Effects of the Invention]

According to an embodiment of the present disclosure, a texture of a medical image may be reconstructed while maintaining a structure of the medical image. By adjusting a texture difference such as a brightness value of a pixel in an image, etc., which varies with a surrounding environment or a photographing device, a medical image may be standardized. The standardized medical image may be used in the field of artificial intelligence, big data, etc.

### [Brief Description of Drawings]

FIG. 1 is a view showing an example of an image reconstruction model according to an embodiment of the present disclosure;
FIGS. 2 and 3 show an example of a detailed configuration of an image reconstruction model according to an embodiment of the present disclosure;
FIG. 4 is a flowchart showing an example of an image reconstruction method according to an embodiment of the present disclosure;
FIGS. 5 and 6 show an example of a training method of an image reconstruction model according to an embodiment of the present disclosure;
FIG. 7 is a block diagram showing a structure of an example of an image reconstruction apparatus according to an embodiment; and
FIG. 8 is a view showing an example of a result of image reconstruction according to an embodiment of the present disclosure.

### [Details for Carrying out the Invention]

Hereinbelow, a method and apparatus for generating an image reconstruction model and a method and apparatus for reconstructing an image by using the image reconstruction model according to an embodiment of the present disclosure will be described in detail.

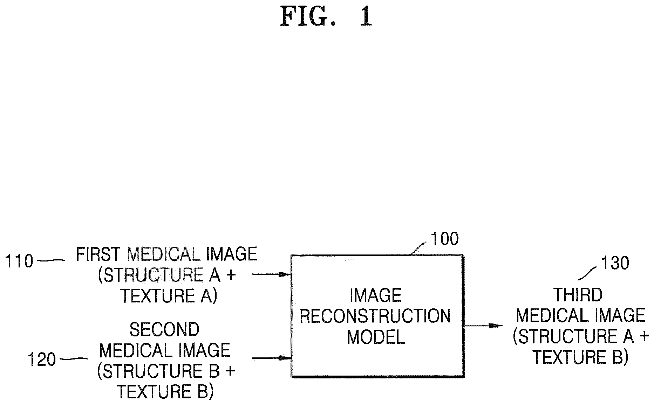

FIG. 1 is a view showing an example of an image reconstruction model according to an embodiment of the present disclosure.

Referring to FIG. 1, an image reconstruction model 100 may be implemented using an artificial neural network that, upon input of a first medical image 110 and a second medical image 120, generates and outputs a third medical image 130 in which features of the two medical images 110 and 120 are combined. That is, the image reconstruction model 110 may be a model that harmonizes the first medical image 110 and the second medical image 120.

The first medical image 110 and the second medical image 120 may be two-dimensional (2D) medical images or three-dimensional (3D) medical images. An example of the 2D medical image may include an X-ray image. An example of the 3D medical image may include a computed tomography (CT) image, a magnetic resonance imaging (MRI) image, an ultrasonic image, etc. The 2D medical images or 3D medical images may be of various types, and they are not limited to specific examples. However, for convenience of description, the description will be made assuming that both the first medical image 110 and the second medical image 120 are X-ray images.

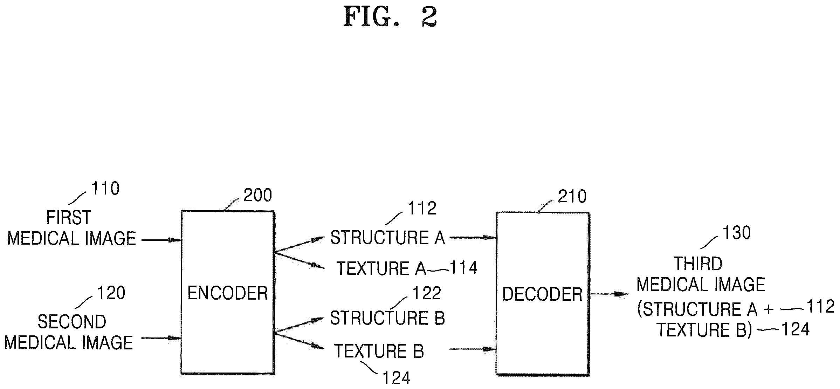

The image reconstruction model 100 may be a model that generates a third medical image 130 in which features of a texture of the second medical image 120 are applied to a structure of the first medical image 110. That is, the image reconstruction model 100 may be a model that reconstructs a texture of the first medical image 110 into the texture of the second medical image 120. Herein, the structure may mean a shape that may be identified in a medical image, and the texture may mean a brightness value, etc., of a pixel that fills an inner side of the shape. For example, the image reconstruction model 100 may be a model that reconstructs a texture, such as a brightness value, etc., of a lung while maintaining a lung shape (i.e., a structure) of the first medical image 110. A detailed method of reconstructing a texture of a medical image into a medical image to which features of a specific texture is applied through the image reconstruction model 100 will be described below with reference to FIG. 2.

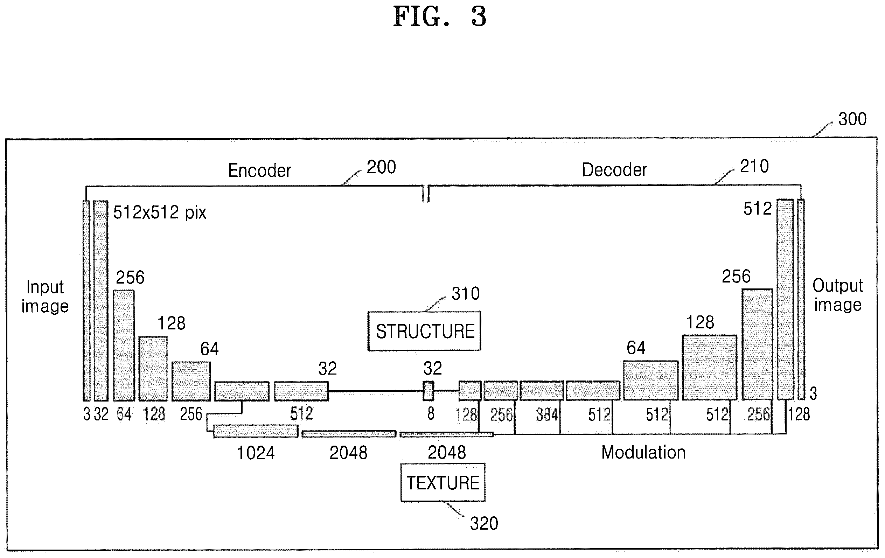

FIGS. 2 and 3 show an example of a detailed configuration of an image reconstruction model according to an embodiment of the present disclosure.

Referring to FIGS. 2 and 3, image reconstruction models 100 and 300 may include an encoder 200 and a decoder 210. The encoder 200 may be an artificial neural network including a plurality of layers for extracting features of a structure 310 and a texture 320 from a medical image. In an embodiment, the encoder 200 may be implemented to extract a structure 310 including a 2D vector of N channels (N is a natural number of 2 or more) (e.g., a 2D vector of a size of 16*16 of 8 channels) and a texture 320 including an one-dimensional (1D) vector of 1 channel (e.g., a 1D vector of a size of 2048) without position information. As the number of channels of the structure increases, the encoder 200 may learn more structural features of a medical image. The number of channels of the structure may change variously according to an embodiment. The decoder 210 may be an artificial neural network including a plurality of layers generating an image based on a structure and a texture.

The encoder 200 may extract a first structure 112 and a first texture 114 from the first medical image 110. The encoder 200 may also extract a second structure 122 and a second texture 124 from the second medical image 120. The decoder 210 may generate the third medical image 130 in which the second texture 124 is applied to the first structure 112, upon input of the first structure 112 of the first medical image 110 and the second texture 124 of the second medical image 120. In another embodiment, the decoder 210 may generate a fourth medical image in which the first texture 114 is applied to the second structure 122 upon input of the second structure 122 of the second medical image 120 and the first texture 114 of the first medical image 110. That is, through the encoder 200 and the decoder 210 of the image reconstruction model 100, a texture may be reconstructed while maintaining a structure of a medical image.

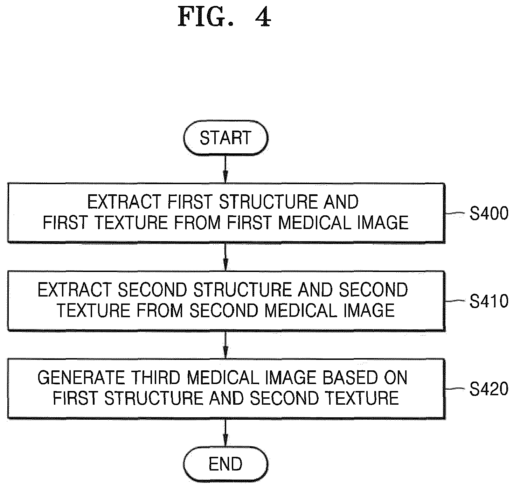

FIG. 4 is a flowchart showing an example of an image reconstruction method according to an embodiment of the present disclosure.

Referring to FIGS. 2 and 4 together, an image reconstruction apparatus may extract the first structure 112 and the first texture 114 from the first medical image 110 through the encoder 200 of the image reconstruction model, in operation S400. The image reconstruction apparatus may extract the second structure 122 and the second texture 124 from the second medical image 120, through the encoder 200, in operation S410. The image reconstruction apparatus may input the first structure 112 of the first medical image 110 and the second texture 124 of the second medical image 120 to the decoder 210 to generate the third medical image 130 in which features of the second texture 124 are applied to the first structure 112, in operation S420.

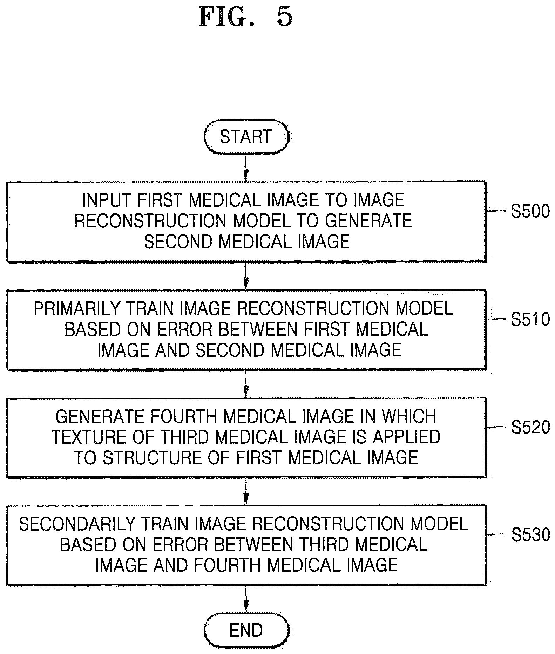

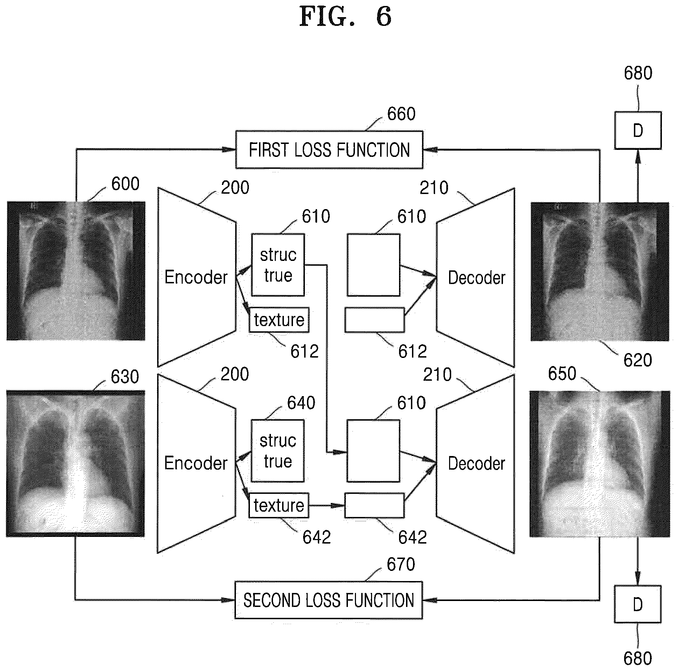

FIGS. 5 and 6 show an example of a training method of an image reconstruction model according to an embodiment of the present disclosure.

Referring to FIGS. 5 and 6 together, the image reconstruction apparatus may input a first medical image 600 to an image reconstruction model to generate a second medical image, in operation S500. For example, the image reconstruction apparatus may input a first structure 610 and a first texture 612 of the first medical image 600 extracted through the encoder 200 to the decoder 210, to generate a second medical image 620. Herein, the first medical image 600 may be an image provided in advance for learning. A plurality of first medical images 600 may be used for learning.

The image reconstruction apparatus may train an image reconstruction model based on a first loss function 660 indicating an error between the first medical image 600 and a second medical image 620, in operation S510. That is, the image reconstruction model may be trained to generate the second medical image 620 that is the same as the first medical image 600. The first loss function 660 may be an L1 loss function indicating a difference between the first medical image 600 and the second medical image 620.

In another embodiment, the image reconstruction apparatus may train the image reconstruction model by using a generative adversarial network (GAN). The encoder 200 and the decoder 220 of the image reconstruction model may correspond to a generator of the GAN. A discriminator D 680 of the GAN may discriminate whether the second medical image 620 is real or fake. A training process of the generator and the discriminator of the GAN is already known widely, and thus will not be further described. Upon completion of training of the generator through the GAN, the generator may be used as the image reconstruction model 100 according to the current embodiment.

The image reconstruction apparatus may further train the image reconstruction model by using two medical images. More specifically, the image reconstruction apparatus may extract features of the second structure 640 and the second texture 642 from the third medical image 630 and generate a fourth medical image 650 in which the features of the second texture 642 of the third medical image 630 are applied to the first structure 610 of the first medical image 600, in operation S520. Herein, the third medical image 630 may be an image provided in advance for learning. The image reconstruction apparatus may train an image reconstruction model based on a second loss function 670 indicating an error between the third medical image 630 and the fourth medical image 640, in operation S530. As the third medical image 630 and the fourth medical image 650 need to have the same features of the textures regardless of their structures, the second loss function 670 may be a function indicating an error between the textures of the two medical images 630 and 640. In an embodiment, the second loss function 670 may be a function indicating a non-saturation loss in which a texture distribution is identified for a plurality of patches randomly extracted from the third medical image 630 and the fourth medical image 650. The size and number of patches may be changed variously according to an embodiment. The non-saturation loss is already known widely and thus will not be described further. In addition, the second loss function 670 of various types for comparing textures of two images may be used in the current embodiment.

In another embodiment, the image reconstruction apparatus may secondarily train the image reconstruction model by using the GAN. The image reconstruction model 110 may correspond to the generator of the GAN as described above. The discriminator 680 of the GAN may discriminate whether the fourth medical image 650 is real or fake. The image reconstruction model may be trained based on the discriminator of the GAN and the second loss function. A training method of the GAN is already known widely and thus will not be described further.

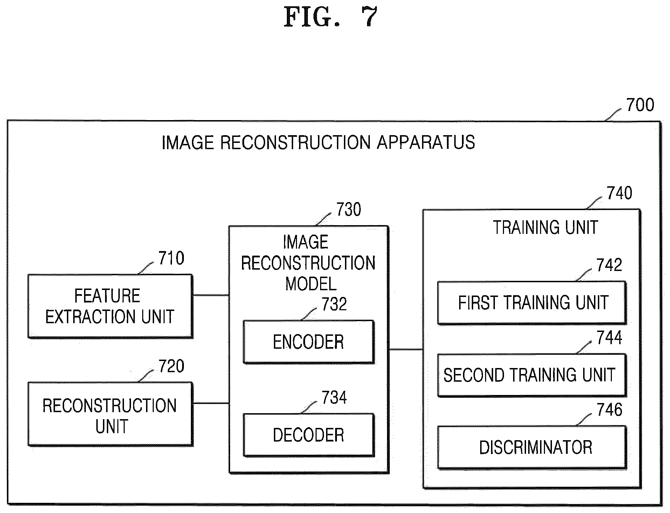

FIG. 7 is a block diagram showing a structure of an example of an image reconstruction apparatus according to an embodiment.

Referring to FIG. 7, an image reconstruction apparatus 700 may include a feature extraction unit 710, a reconstruction unit 720, an image reconstruction model 730, and a training unit 740. In an embodiment, upon completion of training of the image reconstruction model 730, the training unit 740 may be omitted. In another embodiment, the image reconstruction apparatus 700 may be implemented as a computing device including a memory, a processor, and an input/output device. In this case, each component may be implemented with software and loaded on the memory, and then may be executed by the processor.

The feature extraction unit 710 may extract a structure and a texture from a medical image through an encoder 732 of the image reconstruction model 730. For example, upon input of a first medical image and a second medical image, the feature extraction unit 710 may extract a first structure and a first texture from the first medical image and extract a second structure and a second texture from the second medical image, through the encoder 732.

The reconstruction unit 720 may generate a medical image that is a combination of the two medical images. For example, if the first structure and the first texture and the second structure and the second texture are extracted from the first medical image and the second medical image through the feature extraction unit 710, the reconstruction unit 720 may generate a third medical image in which features of the second texture are applied to the first structure through the decoder 734 or a fourth medical image in which features of the first texture are applied to the second structure.

The training unit 740 may train the image reconstruction model 730. According to an embodiment, the training unit 740 may include all or any one of a first training unit 742 and a second training unit 744. If the training unit 740 uses a GAN, the training unit 740 may further include a discriminator 746. Hereinbelow, a description will be made assuming that the training unit 740 includes all of the first training unit 742, the second training unit 744, and the discriminator 746.

The first training unit 742 may input a first training image to the encoder 732 of the image reconstruction model 730 to extract features of a structure and a texture for the first training image. After the first training unit 742 extracts the features, the first training unit 742 may input the structure and the texture of the first training image to the decoder 734 to generate a reconstructed image. As the structure and the texture of the first training image are input to the decoder 734, the image reconstruction model may be trained such that the reconstructed image is the same as the first training image. That is, the first training unit 742 may train the image reconstruction model based on a first loss function indicating an error between the first training image and the reconstructed image. The first loss function may be an L1 loss between the first training image and the reconstructed image.

The second training unit 744 may train the image reconstruction model by using the first training image and the second training image. First, the second training unit 744 may input the first training image and the second training image to the encoder 732 to extract a first structure and a first texture for the first training image and a second structure and a second texture for the second training image. The second training unit 744 may input the first structure and the second texture to the decoder 734 to generate a reconstructed image. As the second texture is a texture feature of the second training image, the second training unit 744 may train the image reconstruction model 730 based on the second loss function indicating whether the texture is correctly applied to the reconstructed image. For example, the second training unit 744 may train the image reconstruction model based on the second loss function indicating a patch-based texture error between the second training image and the reconstructed image. For example, the second loss function may be a function indicating a patch-based non-saturation loss.

In another embodiment, the first training unit 742 and the second training unit 744 may train the image reconstruction model 730 based on a GAN. The first training unit 742 and the second training unit 744 may train the image reconstruction model 730 in a conventional GAN training manner by replacing the generator of the GAN with the image reconstruction model 730. For example, the first training unit 742 may train the generator considering both a discrimination result output by the discriminator 746 of the GAN and the first loss function, and the second training unit 744 may train the generator considering both the discrimination result output by the discriminator 746 of the GAN and the second loss function. After completion of training of the first training unit 742 and the second training unit 744, the generator of the GAN may be used as the image reconstruction model 730 according to the current embodiment.

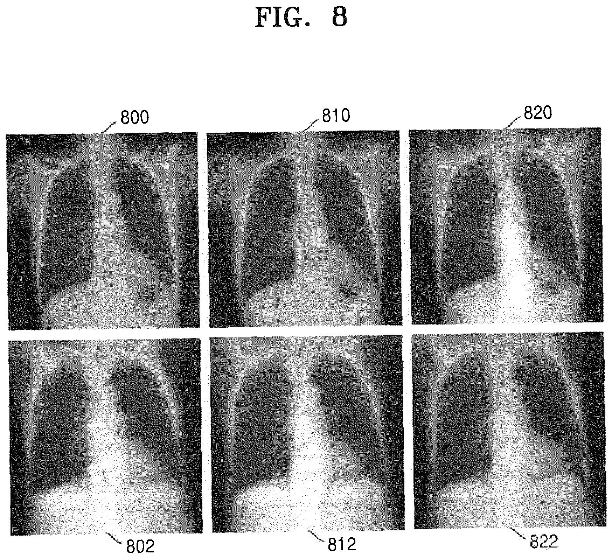

FIG. 8 is a view showing an example of a result of image reconstruction according to an embodiment of the present disclosure.

Referring to FIG. 8, upon input of first medical images 800 and 802 and second medical images 810 and 812, the image reconstruction apparatus 700 may generate third medical images 820 and 822 in which features of textures of the second medical images 810 and 812 are applied to structures of the first medical images 800 and 802. The image reconstruction apparatus 700 may reconstruct only a texture while maintaining a structure of a medical image.

The present disclosure may also be implemented as a computer-readable program code on a computer-readable recording medium. The computer-readable recording medium may include all types of recording devices in which data that is readable by a computer system is stored. Examples of the computer-readable recording medium may include read-only memory (ROM), random access memory (RAM), compact-disc ROM (CD-ROM), a magnetic tape, a floppy disk, an optical data storage device, etc. The computer-readable recording medium may be distributed over computer systems connected through a network to store and execute a computer-readable code in a distributed manner.

So far, embodiments have been described for the present disclosure. It would be understood by those of ordinary skill in the art that the present disclosure may be implemented in a modified form within a scope without departing from the essential characteristics of the present disclosure. Therefore, the disclosed embodiments should be considered in a descriptive sense rather than a restrictive sense. The scope of the present specification is not described above, but in the claims, and all the differences in a range equivalent thereto should be interpreted as being included in the present disclosure.

## Claims

1. A method, performed by an image reconstruction apparatus, of reconstructing an image, the method comprising:
extracting, through an encoder configured to extract a structure and a texture from an image, a first structure and a first texture from a first medical image;
extracting, through the encoder, a second structure and a second texture of a second medical image; and
inputting the first structure of the first medical image and the second texture of the second medical image to a decoder configured to reconstruct an image based on a structure and a texture and generate a third medical image.

2. The method as claimed in claim 1, wherein the encoder comprises an artificial neural network configured to output a structure comprising a two-dimensional (2D) vector of N channels and a texture comprising one-dimensional (1D) vector of 1 channel, wherein N is a natural number of at least 2, and
the decoder comprises an artificial neural network configured to generate an image based on the structure of the 2D vector of the N channels and the texture of the 1D vector of the 1 channel.

3. The method as claimed in claim 1, wherein the first medical image and the second medical image are X-ray images.

4. A method of generating an image reconstruction model, the method comprising:
inputting a first medical image to an image reconstruction model comprising an encoder configured to extract a structure and a texture from an image and a decoder configured to reconstruct the image based on the structure and the texture and generate a second medical image; and
primarily training the image reconstruction model based on a first loss function indicating an error between the first medical image and the second medical image.

5. The method as claimed in claim 4, wherein the primarily training comprises:
discriminating, through a discriminator configured to distinguish between real or fake, whether the second medical image is real or fake; and
training the image reconstruction model by using a discrimination result output by the discriminator and the first loss function.

6. The method as claimed in claim 4, further comprising:
extracting, through the encoder, a first structure and a first texture of the first medical image;
extracting, through the encoder, a second structure and a second texture of a third medical image;
inputting the first structure of the first medical image and the second texture of the third medical image to the decoder to generate a fourth medical image; and
secondarily training the image reconstruction model by using a second loss function indicating a patch-based error between the third medical image and the fourth medical image.

7. The method as claimed in claim 6, wherein the secondarily training comprises:
discriminating whether the fourth medical image is real of fake through a discriminator configured to distinguish between real or fake; and
training the image reconstruction model by using a discrimination result output by the discriminator and the second loss function.

8. An image reconstruction apparatus comprising:
a feature extraction unit configured to extract, through an encoder configured to extract a structure and a texture, a structure and a texture from each of a first medical image and a second medical image; and
a reconstruction unit configured to generate, through a decoder configured to reconstruct an image based on a structure and a texture, a third medical image in which the texture of the second medical image is applied to the structure of the first medical image.

9. The image reconstruction apparatus as claimed in claim 8, further comprising a training unit configured to train an image reconstruction model comprising the encoder and the decoder,
wherein the training unit comprises:
a first training unit configured to train the image reconstruction model based on an error between the first medical image and a fourth medical image obtained by inputting the first medical image to the image reconstruction model; and
a second training unit configured to train the image reconstruction model based on a patch-based error between the second medical image and the third medical image.

10. The image reconstruction apparatus as claimed in claim 9, wherein the training unit further comprises a discriminator configured to discriminate a real medical image from a fake medical image reconstructed by the image reconstruction model, and
the first training unit and/or the second training unit are/is configured to train the image reconstruction model by using a discrimination result output by the discriminator.

11. A computer-readable recording medium having recorded thereon a computer program for executing the method as claimed in claim 1 or claim 4.
